(19) Europäisches Patentamt
European Patent Office
Office européen des brevets

(11) Publication number : 0 643 059 A1

(12) **EUROPEAN PATENT APPLICATION**

(21) Application number : 94306370.1

(22) Date of filing : 30.08.94

(51) Int. Cl.[6] : C07D 401/04, A61K 31/44

(30) Priority : 15.09.93 GB 9319100

(43) Date of publication of application : 15.03.95 Bulletin 95/11

(84) Designated Contracting States : AT BE CH DE DK ES FR GB GR IE IT LI LU NL PT SE

(71) Applicant : Pfizer Limited
Ramsgate Road
Sandwich Kent CT13 9NJ (GB)
(84) GB

(71) Applicant : Pfizer Research and Development Company, N.V./S.A.
Alexandra House,
Earlsfort Centre,
Earlsfort Terrace
Dublin 2 (IE)
(84) BE CH DE DK ES FR GR IE IT LI LU NL PT SE AT

(72) Inventor : Dack, Kevin N., c/o Pfizer Central Res.
Ramsgate Road,
Sandwich
Kent, CT13 9NJ (GB)
Inventor : Dickinson, Roger P., c/o Pfizer Central Res.
Ramsgate Road,
Sandwich
Kent, CT13 9NJ (GB)

(74) Representative : Wood, David John
PFIZER LIMITED,
Ramsgate Road
Sandwich, Kent CT13 9NJ (GB)

(54) **3-(3-Pyridinyl)-1H-indoles as thromboxane A2 synthetase inhibitors.**

(57) 3-(3-Pyridinyl)-1H-indole thromboxane synthetase inhibitors of the formula :-

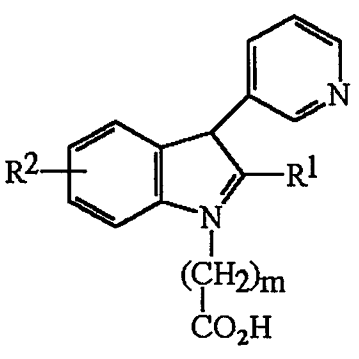

---(I)

or a biolabile ester thereof, or a pharmaceutically acceptable salt of either the compound or of the said biolabile ester, wherein :

$R^1$ is H or $C_1$-$C_4$ alkyl ;
$R_2$ is H, $C_1$-$C_4$ alkyl, $C_1$-$C_4$ alkoxy or halo ; and
when $R^1$ is H, m is 4, and when $R^1$ is $C_1$-$C_4$ alkyl, m is 2, 3 or 4.

EP 0 643 059 A1

This invention relates to certain substituted 3-(3-pyridinyl)-1H-indoles which selectively inhibit the thromboxane $A_2$ synthetase enzyme without significantly inhibiting the action of the prostacyclin synthetase or cyclooxygenase enzymes. The compounds are thus useful as therapeutic agents, preferably in combination with a thromboxane $A_2$/endoperoxide receptor antagonist, for example in the treatment of atherosclerosis and unstable angina and for prevention of reocclusion, both acute and chronic, after percutaneous transluminal coronary and femoral angioplasty. They may also find clinical utility in a further variety of disease conditions in which thromboxane $A_2$ has been implicated such as in the treatment of myocardial infarction, stroke, cardiac arrhythmias, transient ischaemic attack, tumour metastasis, peripheral vascular disease, bronchial asthma, renal disease, cyclosporin-induced nephrotoxicity, renal allograft rejection, trauma, vascular complications of diabetes and endotoxin shock, and in coronary artery bypass surgery and haemodialysis.

Clinical evaluation of thromboxane synthetase inhibitors has, in general, been disappointing, and several reasons have been put forward to explain the poor performance. It is now realised that prostaglandin $H_2$, the precursor to thromboxane $A_2$, can also activate the thromboxane receptor thus negating the potential beneficial effect of enzyme inhibition. This problem can be overcome by combining the thromboxane synthetase inhibitor with an antagonist of the thromboxane $A_2$/prostaglandin $H_2$ receptor. In addition, there is evidence to suggest that complete inhibition of thromboxane synthetase is important, and doses of inhibitors used may have been insufficient to achieve this. Compounds with greater potency would therefore be desirable.

The potency of thromboxane synthetase inhibitors is usually expressed as the concentration of compound required to inhibit platelet microsomal thromboxane synthetase by 50% ($IC_{50}$). When thromboxane synthetase inhibitors are used clinically, the potency in human whole blood is clearly of more importance than that in an isolated enzyme assay. Furthermore, the concentration required to inhibit the enzyme by at least 80% in whole blood is probably a more relevant predictor of the ability of the compound to produce complete inhibition of thromboxane synthesis in humans.

Surprisingly, it has been found that compounds of the present invention have a lower $IC_{80}$ in human whole blood than pyridyl-substituted indole $TxA_2$ synthetase inhibitors reported previously, with the result that relatively low doses are generally required in humans to achieve a sustained high level of inhibition of thromboxane synthesis.

The compounds of the invention have the formula:

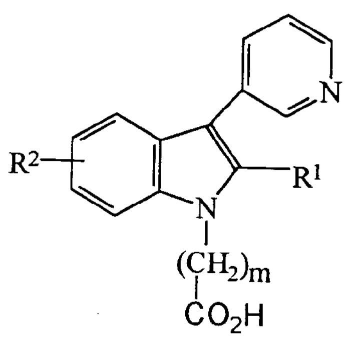

---(I)

and include biolabile esters thereof and pharmaceutically acceptable salts both of (I) and of the said biolabile esters wherein:

$R^1$ is H or $C_1$-$C_4$ alkyl;

$R_2$ is H, $C_1$-$C_4$ alkyl, $C_1$-$C_4$ alkoxy or halo; and

when $R^1$ is H, m is 4, and when $R^1$ is $C_1$-$C_4$ alkyl, m is 2, 3 or 4.

Alkyl and alkoxy groups having three or more carbon atoms may be straight- or branched-chain. "Halo" means fluoro, chloro, bromo or iodo.

Also included in the invention are radiolabelled derivatives of compounds of formula (I) which are suitable for biological studies.

The term biolabile ester in the above definition means a pharmaceutically acceptable, biologically degradable ester derivative of a compound of formula (I), that is a prodrug which, upon administration to an animal or human being, is converted in the body to a compound of the formula (I).

In the case of the compounds of formula (I), such biolabile ester prodrugs are particularly advantageous in providing compounds of formula (I) suitable for oral administration. The suitability of any particular ester-forming group can be assessed by conventional in vivo animal or in vitro enzyme hydrolysis studies. Thus desirably, for optimum effect, the ester should only be hydrolysed after absorption is complete. Accordingly, the

ester should be resistant to premature hydrolysis by digestive enzymes before absorption, but should be productively hydrolysed by, for example, gut-wall, plasma or liver enzymes. In this way, the active acid is released into the bloodstream following oral absorption of the prodrug.

Suitable biolabile esters have the formula(IA):-

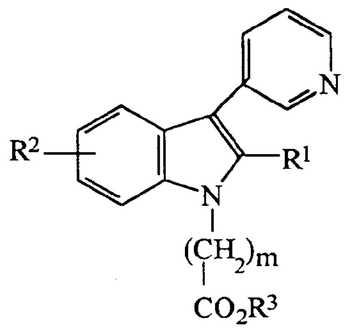

---(IA)

in which $R^1$, $R^2$ and m are as defined for formula (I) and $R^3$ includes alkyl,alkanoyloxyalkyl, cycloalkanoyloxyalkyl, aroyloxyalkyl and alkoxycarbonyloxyalkyl, including cycloalkyl and aryl substituted derivatives thereof, aryl and cycloalkyl, wherein said alkyl, alkanoyl and alkoxy groups contain up to 8 carbon atoms and can be branched-chain or straight-chain, said cycloalkyl groups contain from 3-7 carbon atoms and said cycloalkanoyl groups from 4-8 carbon atoms wherein both are optionally benzo-fused, and said aryl and aroyl group include optionally substituted phenyl, naphthyl and indanyl ring systems. These esters are also believed to have some activity as thromboxane $A_2$ synthetase inhibitors in their own right.

Examples of biolabile ester groups are ethyl, indanyl, isopropyl, n-butyl, sec-butyl, t-butyl, cyclohexyl, benzyl, phenethyl, phenpropyl, acetonyl, glyceryl, pivaloyloxymethyl, 5-(4-methyl-1,3-dioxolene-2-only)methyl, cyclohexylmethyl, cyclohexylcarboxyethyl, cyclohexylacetoxyethyl, propionyloxyisobutyl, hexanoyloxyethyl, pentanoyloxyethyl, acetoxyethyl, acetoxybenzyl, pentanoyloxybenzyl, cyclohexyloxycarbonyloxyethyl, butyloxycarbonyloxyethyl, isobutyloxycarbonylethyl and ethoxycarbonyloxyethyl.

Preferably, the biolabile esters of the invention are $C_1$-$C_4$ alkyl esters (Thus $R^3$ is preferably $C_1$-$C_4$ alkyl). More preferably, they are methyl, ethyl or t-butyl esters.

The pharmaceutically acceptable salts of the compounds of formula (I) are those formed with bases or acids which provide non-toxic salts. Examples of the former include the alkali and alkaline earth metal salts such as the sodium, potassium or calcium salts, and salts with amines such as diethylamine. Examples of pharmaceutically acceptable acid addition salts include the hydrochloride, hydrobromide, sulphate or bisulphate, phosphate or hydrogen phosphate, methanesulphonate, benzenesulphonate and p-toluenesulphonate.

The preferred alkyl groups are methyl and ethyl, and the preferred alkoxy groups are methoxy and ethoxy.

$R^1$ is preferably H or methyl.

$R^2$, when a substituent, is preferably in the 5-position.

$R^2$ is preferably H, $C_1$-$C_4$ alkoxy or halo, most preferably H, methoxy or chloro.

m is preferably 4.

In another aspect the present invention provides processes for the preparation of compounds of formulae (I), biolabile esters thereof, and pharmaceutically acceptable salts of these.

The acids of the formula (I) can be obtained by hydrolysis of their biolabile ester precursors of the formula (IA):-

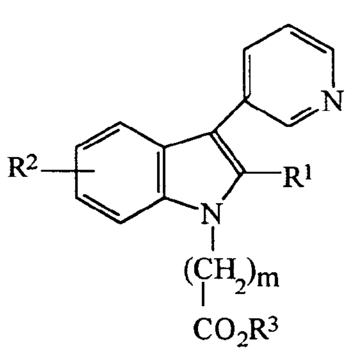

---(IA)

wherein $R^1$, $R^2$, $R^3$ and m are as defined for formula (IA). $R^3$ in this process is preferably $C_1$-$C_4$ alkyl most preferably methyl, ethyl or t-butyl.

The reaction can be conducted under either basic or acidic conditions, e.g. using excess aqueous alkali metal hydroxide solution, preferably sodium hydroxide solution, or excess hydrochloric acid, respectively, optionally with an organic co-solvent such as a $C_1$-$C_4$ alkanol, preferably methanol, at from about 20°C up to the reflux temperature of the reaction mixture.

Depending on the nature of m, the compounds of formula (IA) can be obtained as described below:-

(A) When m is 3 or 4, the compounds of the formula (IA) can be obtained by reaction of the indole precursors(II):-

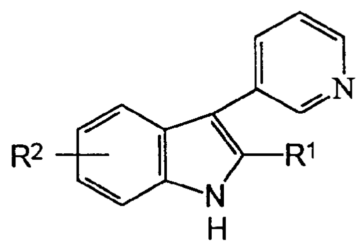

(II)

where $R^1$ and $R^2$ are as defined for formula (I), with an ester of the formula:-

$$X.(CH_2)_mCO_2R^3$$

where $R^3$ is as defined for formula (IA), m is 3 or 4, and X is a leaving group, preferably halo and most preferably bromo, in the presence of a base, preferably sodium hydride, typically in a suitable dry organic solvent, preferably dry dimethylformamide, at from 0° to 100°C.

(B) When m is 2, the compounds of the formula (IA) can be obtained by the Michael addition of an indole precursor of the formula (II) as defined in (A) with an alkenoate ester of the formula (III) in the presence of a base, preferably benzyltrimethylammonium hydroxide or tetrabutylammonium fluoride (or $C_1$-$C_4$ alkoxide), typically in a suitable organic solvent such as 1,4-dioxane or tetrahydrofuran at from 0° to 100°C. Room temperature is often suitable.

$$CH_2 = CH.CO_2R^3 \qquad (III)$$

$R^3$ is as defined for formula (IA).

The indoles (II) can be prepared by the Fischer indole synthesis using an appropriately substituted phenylhydrazine (IV) with either a 3-pyridinyl methylketone (V) when $R^1$ is $C_1$-$C_4$ alkyl, or 3-pyridinylacetaldehyde or (preferably) a protected enol ether form such as (VI) when $R^1$ is H.

The Fischer indole synthesis can be achieved under acidic conditions such as heating in hydrochloric acid, optionally with a co-solvent such as acetic acid, or any of the published procedures known to those skilled in the art.

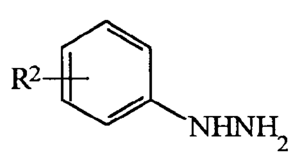

(IV)

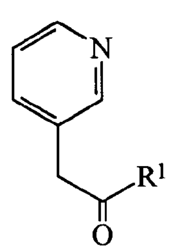

(V)

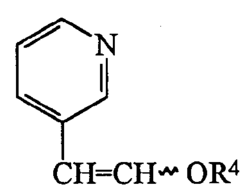

(VI)

[$R^4$ = $C_1$-$C_4$ alkyl; the cis and/or trans form can be used] Many of the phenyl hydrazines are commercially available, and the ketones (V) and enol ethers (VI) are readily prepared by conventional techniques such as those described in the following "Preparations" section.

The biological activity of the compounds of the invention has been demonstrated using the following in vitro and in vivo assay procedures:

1. Cyclo-oxygenase

Ram seminal vesicle microsomes (Biochemistry, 1971, 10, 2372) are incubated with arachidonic acid (100.0μM) for 1.0 minute at 22°C to produce prostaglandin endoperoxide ($PGH_2$). Aliquots of $PGH_2$ are incubated for 30 seconds at 22°C with pig aorta microsomes (Nature, 1976, 263, 663) and the reaction terminated with five volumes of ethanol. $PGI_2$ production is assessed by measuring its stable breakdown product 6-keto-$PGF_{1\alpha}$, using a specific radioimmunoassay.

The test compound is pre-incubated with the cyclo-oxygenase source in ram seminal vesicles for 30 minutes at 0°C. The ability of the compound to inhibit $PGH_2$ formation by the enzyme is measured indirectly by evaluation of the 6-keto-$PGF_{1\alpha}$ produced upon addition of $PGI_2$ synthetase (30 seconds at 22°C).

2. Prostacyclin ($PGI_2$) Synthetase

Pig aorta microsomes (Nature, 1976, 263, 663) are incubated for 30 seconds at 22°C with $PGH_2$ (produced as in 1) and the reaction terminated with 5 volumes of ethanol. $PGI_2$ production is assessed by measuring its stable breakdown product, 6-keto $PGF_{1\alpha}$, using a specific radioimmunoassay. $PGI_2$ production can be completely inhibited by pre-incubation of the enzyme $PGI_2$ synthetase with the selective $PGI_2$ synthetase inhibitor 15-hydroperoxyarachidonic acid (Prostaglandins, 1976, 12, 715). The test compound is pre-incubated with the enzyme for 5 minutes, and its ability to prevent the production of $PGI_2$ (6-keto-$PGF_{1\alpha}$) is measured.

3. Thromboxane $A_2$ ($TxA_2$) Synthetase

Indomethacin pretreated human platelet microsomes (Science 1976, 193, 163) are incubated for 2 minutes at 0°C with $PGH_2$ (produced as in 1) and the reaction terminated with 5 volumes of ethanol. $TxA_2$ production is assessed by measuring its stable metabolite $TxB_2$, using a specific radioimmunoassay.

The test compound is pre-incubated with enzyme for 5 minutes, and its ability to inhibit the thromboxane $A_2$ synthetase enzyme is measured as the reduction os $TxA_2$ ($TxB_2$) production.

Compounds of the formula (I) tested in this way have been shown to be capable of selectively inhibiting the thromboxane $A_2$ synthetase enzyme.

4. Human Blood $TxA_2$Synthetase Inhibition

Approximately 20ml blood is taken from the antecubital vein of volunteers (ensuring they have not used any medication, especially aspirin, in the previous two weeks), into a sterile syringe which is immediately dispensed in lml aliquots into glass vials containing the test compound or vehicle (50μl) at a range of concentrations (0.03-10μM). Each vial is mixed gently for 5 seconds and then incubated in a water bath at 37°C for 2 hours.

The serum is removed and spun at 14000rpm for 2 min. The supernatant is then frozen at -20° prior to radioimmunoassay.

The supernatant samples are ethanol extracted (1:6), then diluted in gel buffer (1:35) to give a final dilution of (1:210). Radioimmunoassay is carried out to determine the level of immunoreactive $TxB_2$ in the samples and this is expressed as % inhibition of control $TxB_2$ levels.

5. Anaesthetised Rabbits

Thromboxane $A_2$ synthetase inhibition is evaluated ex vivo in anaesthetised rabbits as follows:

New Zealand White rabbits (2-2.5 kg) are anaesthetised with fetanyl citrate (0.189 mg) and fluanisone (6 mg) intramuscularly and midazolam (3 mg) intravenously and maintained by an intravenous infusion of fentanyl citrate (0.315 mg), fluanisone (1 mg) and midazolam (1 mg) per hour. After cannulation of the trachea, a carotid artery is cannulated for collection of blood samples. The catheter is kept patent by the presence within the catheter of saline containing heparin (50μ/ml). Control carotid arterial blood samples are taken 25 and 5 minutes prior to administration of the test compound via a marginal ear vein. Two groups of rabbits are used. The first group received 0.01 mg/kg of the test compound followed, at ten minute intervals, by 0.03, 0.1, 0.3, 1.0, 3.0 and 10 mg/kg; the second group comprises the controls. Carotid arterial blood samples are taken 5 minutes after all doses. At each time point, a 1 ml blood sample is collected in a glass test tube, without anticoagulant, for $TxB_2$ determination. This blood is allowed to clot during a two hour incubation at 37°C and the serum obtained by centrifugation. Serum samples are then processed through the $TxB_2$ radioimmunoassay after deproteinisation with ethanol.

6. Conscious Dogs

Thromboxane $A_2$ synthetase inhibition may also be evaluated ex vivo in sling-restrained conscious dogs after oral (p.o.) or intravenous (i.v.) administration of a compound of the invention. The sampling and assaying procedures employed are similar to those described for the ex vivo anaesthetised rabbit experiments.

For administration to man, in the therapy or prevention of diseases or adverse medical conditions in which $TxA_2$ is implicated as a causative agent, oral dosages of the compounds would be expected to be in the range of from 10-400 mg daily for an average adult patient (70 kg). Thus for a typical adult patient, individual tablets or capsules contain from 10 to 400 mg of active compound, in a suitable pharmaceutically acceptable vehicle or carrier, for administration as a single dose, or in multiple doses, once or several times a day. Dosages for intravenous administration would typically be within the range of from 5 to 400 mg per single dose required. In practice the physician will determine the actual dosage which will be most suitable for an individual patient and it will vary with the age, weight and response of the particular patient, and with the condition being treated. The above dosages are exemplary of the average case but there can, of course, be individual instances where higher or lower dosage ranges are merited, and such are within the scope of this invention.

For human use, the compounds of the formula (I) can be either administered alone or in combination with a thromboxane $A_2$/endoperoxide receptor antagonist (such as:-
3-[(4-fluorophenyl)methyl]-5-[2-[(4-trifluoromethylphenyl)sulphonylamino]ethyl]benzenepropanoic acid;
3-[2-[(4-chlorophenyl)sulphonylamino]ethyl]-5-[1-(4-fluorophenyl)ethyl]benzenepropanoic acid;
3-[2-[(4-chlorophenyl)sulphonylamino]ethyl]-5-[(4-fluorophenyl)methyl]benzenepropanoic acid;
3-[2-[(4-chlorophenyl)sulphonylamino]ethyl]-5-(4-fluorophenoxy)benzenepropanoic acid;
3-[2-[(4-chlorophenyl)sulphonylamino]ethyl]-5-(4-methoxybenzoyl)benzenepropanoic acid; or
3-[2-[(4-chlorophenyl)sulphonylamino]ethyl]-5-(4-cyanobenzoyl)benzenepropanoic acid), but will generally be administered in admixture with a pharmaceutical carrier selected with regard to the intended route of administration and standard pharmaceutical practice. For example, they may be administered orally in the form of tablets containing such excipients as starch or lactose, or in capsules or ovules either alone or in admixture with excipients, or in the form of elixirs or suspensions containing flavouring or colouring agents. They may be injected parenterally, for example, intravenously, intramuscularly or subcutaneously. For parenteral administration, they are best used in the form of a sterile aqueous solution which may contain other substances, for example enough salts or glucose, to make the solution isotonic with blood.

Thus the invention provides a pharmaceutical composition comprising a compound of formula (I), or a biolabile ester thereof, or a pharmaceutically acceptable salt of either, together with a pharmaceutically acceptable diluent or carrier, and optionally with a thromboxane $A_2$/endoperoxide receptor antagonist.

The invention also provides a compound of formula (I), or a biolabile ester thereof, or a pharmaceutically acceptable salt of either, or a pharmaceutical composition containing any of these entities, for use in medicine.

The invention further includes the use of a compound of formula (I), or a biolabile ester thereof, or a pharmaceutically acceptable salt of either, or a pharmaceutical composition containing any of these entities, for the manufacture of a medicament for the treatment of disease conditions in which thromboxane $A_2$ is a causative agent.

In a further aspect, the invention provides a method of treating or preventing disease conditions in which thromboxane $A_2$ is a causative agent in a mammal (including a human being) which comprises administering to said mammal a therapeutically effective amount of a compound of formula (I), or a biolabile ester thereof, or a pharmaceutically acceptable salt of either, or a pharmaceutical composition containing any of these entities.

The invention also includes any novel intermediates disclosed herein such as those of formula (II).

The synthesis of the compounds of the invention and of intermediates for use in their preparation are illustrated by the following Examples and Preparations. The purity of the compounds was routinely monitored by thin layer chromatography (TLC) using Merck "Kieselgel 60" $F_{254}$ plates and the following solvent systems (SS):

"SS 1": dichloromethane : methanol, 95:5.
"SS 2": dichloromethane : methanol, 98:2.
"SS 3": hexane : ethyl acetate, 50:50.

$^1$H-Nuclear magnetic resonance (NMR) spectra were recorded using either a Nicolet QE-300 or a Bruker AC-300 spectrometer and were in all cases consistent with the proposed structures. Chemical shifts are given in parts-per-million downfield from tetramethylsilane using conventional abbreviations for designation of major peaks : s, singlet; d, doublet; t, triplet; m, multiplet and br, broad.

EXAMPLE 1

5-(2-Methyl-3-[3-pyridinyl]-1H-indol-l-yl)pentanoic acid.

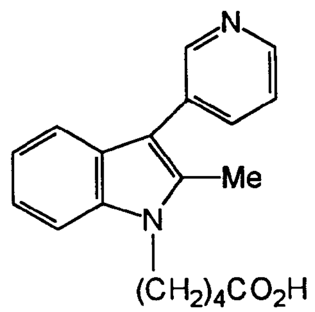

A mixture of methanol (3ml), IN aqueous sodium hydroxide solution (10ml) and ethyl 5-(2-methyl-3-[3-pyridinyl]-1H-indol-1-yl)pentanoate (Example 6,1.0g) was heated at reflux for 2 hours; an homogenous solution was obtained after 3/4 hour. The solvent was evaporated under vacuum and the residue dissolved in water. The resulting solution was washed with ethyl acetate and then acidified to pH 4-5 with glacial acetic acid. The resulting gum was extracted with ethyl acetate and the solution was dried over $Na_2SO_4$ and filtered. Evaporation of solvent under vacuum gave a gum which crystallised on scratching under diethyl ether. This solid was collected by filtration, washed with diethyl ether and dried, to afford the title compound (607mg), m.p. 113-115°C.

Analysis %:

Found : C,73.68; H,6.43; N,9.15;

Calculated for $C_{19}H_{20}N_2O_2$ : C,74.00; H,6.54; N,9.09%

The following compounds were obtained by hydrolysis of their corresponding alkyl esters by a procedure similar to that described in Example 1.

EXAMPLES 2-5

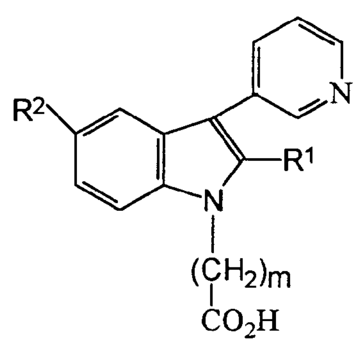

| Example No. | $R^1$ | $R^2$ | m | Analysis % (Theoretical in brackets) C H N | m.p. (°C) |
|---|---|---|---|---|---|
| 2 | Me | Cl | 4 | 66.55 5.64 7.69 (66.26 5.68 7.97)‡ | 165 - 166 |
| 3 | Me | -OMe | 4 | 69.52 6.57 7.98 (67.14 6.67 8.07)* | 169 - 170 |
| 4 | H | H | 4 | 73.46 6.04 9.51 (73.44 6.16 9.52) | 108 - 110 |
| 5 | Me | H | 2 | 72.71 5.45 9.81 (72.83 5.75 9.99) | 130 - 131 |

‡ As 1/10 ethyl acetate
* As hemihydrate

EXAMPLE 6

Ethyl 5-(2-Methyl-3-[3-pyridinyl]-1H-indol-l-yl)pentanoate.

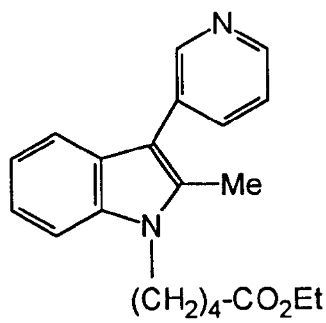

Solid sodium hydride (50% dispersion in mineral oil; 253mg) was added to a stirred solution of 2-methyl-3-(3-pyridinyl)-1H-indole(Preparation 2; 1g) in dry dimethylformamide (10ml) at room temperature under an atmosphere of dry nitrogen. This mixture was stirred for 25 minutes and then ethyl 5-bromopentanoate (1.1g) was added. Stirring was continued for 3 hours at room temperature and then the mixture was poured into water and extracted with ethyl acetate (x3). The combined extracts were washed with water, dried over $Na_2SO4$ and filtered. The solvent was evaporated under vacuum and the residue was chromotographed on silica gel using dichloromethane: methanol (99:1) mixture as the eluent. The product-containing fractions were combined and evaporated under vacuum to give the title compound as a viscous oil, Rf 0.4 ("ss 1").

Found: C,75.33; H, 7.41; N, 8.58

Calculated for $C_{21}H_{24}N_2O_2$: C, 74.97; H, 7.19; N,8.33%

The following compounds were obtained from their respective indoles and bromoalkanoate esters by a procedure similar to that described in Example 6.

EXAMPLES 7-9

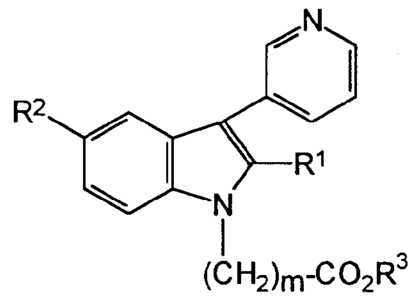

| Example No. | $R^1$ | $R^2$ | $R^3$ | m | Analysis % (Theoretical in brackets) C H N | tlc |
|---|---|---|---|---|---|---|
| 7 | Me | Cl | Et | 4 | 65.84 6.25 6.98 (65.65 6.08 7.22)‡ | Viscous oil Rf 0.2 (ss 2) |
| 8 | Me | -OMe | Et | 4 | 71.57 7.19 7.41 (72.10 7.15 7.64) | Viscous oil Rf 0.11 (ss 2) |
| 9 | H | H | Et | 4 | 74.53 6.78 8.69 (74.50 6.88 8.69) | Viscous gum Rf 0.32 (ss 1) |

‡ 1/5 $CH_2Cl_2$

EXAMPLE 10

Methyl 3-(2-methyl-3-[3-pyridinyl]-1H-indol-l-yl)propanoate

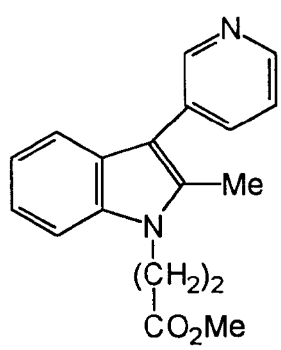

Potassium t-butoxide (107mg) was added to a stirred solution of 2-methyl-3-(3-pyridinyl)-1H-indole (Preparation 2, 1.42g), tetra-n-butylammonium bromide (30mg) and methyl propenoate (0.86ml) in a mixture of dry tetrahydrofuran (13ml) and t-butanol (1.4ml) at room temperature, and the resultant solution was stirred for

24 hours. This solution was poured into water and extracted with ethyl acetate, dried over $MgSO_4$, filtered and evaporated under vacuum. The resultant residue was chromatographed on silica gel using ethyl acetate:hexane (1:3 through to 2:1) gradient mixture as eluent. The product-containing fractions were combined and evaporated under vacuum. The residue was re-chromatographed on silica gel using dichloromethane:methanol (99:1 then 98:2) mixture as eluent. The product-containing fractions were combined and evaporated under vacuum to afford the title compound as a viscous oil (1.35g), Rf 0.15 (ss 3).
N.M.R. $\delta$ ($CDCl_3$): 2.52(3H,s), 2.90(2H,t,J=6.7Hz), 3.72(3H,s), 4.53(2H,t,J=6.7Hz), 7.15(1H,t), 7.25(1H,m), 7.40(2H,m), 7.62(1H,d,J=8.5 Hz), 7.85(1H,m), 8.55(1H,m), 8.78(1H,d,J=1.9Hz).

EXAMPLE 11

Pharmaceutical capsules.

| | mg./capsule |
|---|---|
| Compound of Example 1 | 50 |
| Thromboxane $A_2$ antagonist | 50 |
| Starch | 149 |
| Magnesium stearate B.P. | 1.0 |
| | 250 mg |

The thromboxane $A_2$ antagonist and the compound of Example 1 are sieved and blended with the starch and the excipients. The mix is filled into size no.2 hard gelatin capsules, using suitable machinery. Capsules of other strengths or with different ratios of active ingredients may be prepared in a similar manner.
The following Preparations illustrate the Preparation of certain starting materials used in the previous Examples:-

PREPARATION 1

3-(3-pyridinyl)-1H-indole
Phenylhydrazine (2.56g) was added to 3-(2-EZ-methoxyethenyl)pyridine(Preparation 5,3.2g) and the mixture was stirred to form an homogeneous paste. Concentrated hydrochloric acid (25ml) was added to this paste, resulting in a large exotherm. When the exotherm had subsided, the mixture was heated at 100°C for 45 minutes and then cooled. The bulk of the hydrochloric acid was then removed by evaporation under vacuum and the resultant residue was dissolved in water. This acidic solution was basified with aqueous potassium hydroxide and extracted with ethyl acetate. The combined extracts were dried over $MgSO_4$ and filtered. The solvent was removed by evaporation under vacuum and the residue was chromatographed on silica gel using a dichloromethane:methanol (98:2) mixture as the eluant. The product-containing fractions were combined and evaporated under vacuum to give a solid which was rechromatographed on silica gel using ethyl acetate:hexane (30:70 then 50:50) gradient mixture as the eluant. The product-containing fractions were combined and evaporated under vacuum to give a solid which was crystallised from dichloromethane/hexane to give the title compound (2.7g), m.p. 153-155°C, Rf 0.25 (ss 1).

Found: C, 80.40; H,5.13; N,14.46;
Calculated for $C_{13}H_{10}N_2$: C, 80.38; H,5.19; N,14.43%.

PREPARATION 2

2-Methyl-3-(3-pyridinyl)-1H-indole
Phenylhydrazine(5.4g) was added to 1-(3-pyridinyl)-2-propanone (Preparation 6,6.75g) and the mixture was stirred to form an homogenous paste of the resultant phenylhydrazone. Glacial acetic acid (20ml) was added to this paste, followed by concentrated hydrochloric acid (100ml). The resultant suspension was heated at 100°C for 3 hours to give an homogenous dark brown solution. The solvent was then removed by evaporation under vacuum and the residue was dissolved in water (100 ml). This acidic solution was basified with ammonium hydroxide solution and extracted with ethyl acetate. The combined extracts were dried over $Na_2SO_4$ and filtered. Evaporation of the solvent under vacuum gave a residue which was chromatographed on silica gel

using dichloromethane:methanol(98.5:1.5) mixture as eluent. The product-containing fractions were evaporated under vacuum and the resultant oil was crystallized by scratching under a diethyl ether:n-hexane(50:50) mixture. The title compound was collected by filtration, washing with n-hexane (5.4g); m.p. 126-127°C, $R_f$ 0.35(ss 1).

Found: C,80.88; H,5.66; N,13.63;

Calculated for $C_{14}H_{12}N_2$: C,80.73; H,5.81; N,13.45%.

The following compounds were obtained from 1-(3-pyridinyl)-2-propanone and the appropriately substituted phenylhydrazine by a procedure similar to that described in Preparation 2.

PREPARATION 3

5-Chloro-2-methyl-3-(3-pyridinyl)-1H-indole,

m.p. 201-202°C, Rf 0.12 (ss 3).

Found: C,69.14; H, 4.40; N, 11.14;

Calculated for $C_{14}H_{11}ClN_2$: C,69.28; H, 4.57; N, 11.54%.

(From 4-chlorophenylhydrazine)

PREPARATION 4

5-Methoxy-2-methyl-3-(3-pyridinyl)-1H-indole

m.p. 152-153°C, Rf 0.15 (ss 3).

Found: C,75.39; H,5.80; N,11.19;

Calculated for $C_{15}H_{14}N_2O$.1/15 ethyl acetate: C,75.11; H,5.99; N,11.48%.

(From 4-methoxyphenylhydrazine)

PREPARATION 5

3-(2-EZ-Methoxyethenyl)pyridine

A 1.8M solution of phenyllithium in diethyl ether (111ml) was added dropwise to a stirred, cooled mixture of (methoxymethyl)-triphenylphosphonium chloride (68.6g) in anhydrous diethyl ether (600ml) at -50°C. Stirring was continued for 2 hours at between -50° and -70°C, and then the mixture was allowed to warm to 0°C over 30 minutes. 3-Pyridinecarboxaldehyde (9.4ml) was added to the mixture, and stirring was continued for 3 hours at 0°C and then for 17 hours at room temperature.

Aqueous ammonium chloride solution was added to the mixture and the diethyl ether layer was separated. The aqueous solution was extracted twice with ethyl acetate. The ethyl acetate and diethyl ether extracts were combined, dried over $MgSO_4$ and filtered. The solvents were evaporated under vacuum and the residue was chromatographed as silica gel using ethyl acetate:hexane (20:80 through to 50:50) gradient mixture as eluent. The product-containing fractions were combined and evaporated under vacuum to afford the title EZ mixture as an oil (8.82g) in a 40:60 ratio.

Major isomer - Rf 0.35 (ss 3)

N.M.R. δ ($CDCl_3$): 3.70(3H,s), 5.75(1H,d,J=14Hz), 7.05(1H,d,J=14Hz), 7.15(1H,m), 7.50(1H,d,J=9Hz), 8.35 (1H,d,J=2Hz), 8.42(1H,brs).

Minor isomer - Rf 0.40 (ss 3)

N.M.R. δ ($CDCl_3$): 3.80(3H,s), 5.20(1H,d,J=8Hz), 6.23(1H,d,J=8Hz), 7.19(1H,m), 7.97(1H,d,J=9Hz), 8.35(1H,d,J=2Hz), 8.62(1H,brs).

PREPARATION 6

1-(3-pyridinyl)-2-propanone

1,1[1]-Carbonyldiimidazole (20.4g) was added to a stirred mixture of 3-pyridinylacetic acid hydrochloride (19.9g) and triethylamine (19ml) in anhydrous dichloromethane (200ml). The stirred mixture was heated to reflux for 1 hour, then cooled to room temperature. 2,2-Dimethyl-1,3-dioxane-4,6-dione(19.8g) and pyridine(50ml) were added to the mixture, and stirring was continued at room temperature for 18 hours. The solvent was evaporated under vacuum and the residue was dissolved in a mixture of 2N hydrochloric acid (150ml), glacial acetic acid (20ml) and concentrated hydrochloric acid (30ml). The red-coloured mixture was heated at reflux until the mixture was decolourised and carbon dioxide evolution had ceased (about 3 hours). The solution was cooled and the solvents were evaporated under vacuum. The residue was basified with 2N sodium hydroxide solution and the aqueous mixture was extracted with ethyl acetate (x4). The combined extracts were dried over $MgSO_4$,

filtered and evaporated under vacuum. The residue was chromatographed on silica gel using dichloromethane:methanol (97:1 then 98:2) mixture as eluent. The product-containing fractions were combined and evaporated under vacuum to afford the title compound as an oil, (9.7g), Rf 0.25 (ss 1).
N.M.R. δ ($CDCl_3$): 2.21(3H,s), 3.71(2H,s), 7.25(1H,m), 7.52(1H,d,J=9Hz), 8.42(1H,br s), 8.53(1H,d,J=3).

## Claims

1. A compound of the formula:

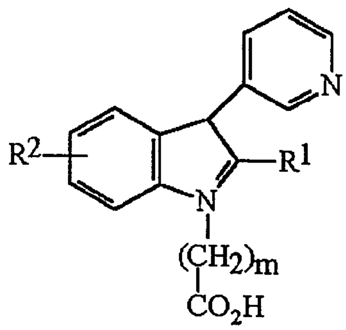

---(I)

or a biolabile ester thereof, or a pharmaceutically acceptable salt of either the compound or of the said biolabile ester, wherein:

$R^1$ is H or $C_1$-$C_4$ alkyl;
$R_2$ is H, $C_1$-$C_4$ alkyl, $C_1$-$C_4$ alkoxy or halo; and
when $R^1$ is H, m is 4, and when $R^1$ is $C_1$-$C_4$ alkyl, m is 2, 3 or 4.

2. A biolabile ester of a compound of the formula (I) as claimed in claim 1, which has the formula:-

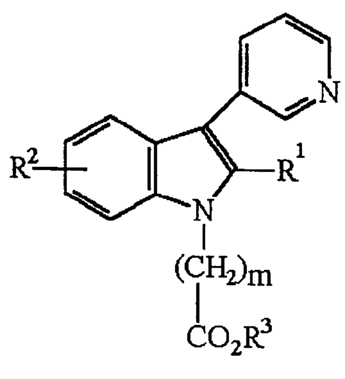

---(IA)

in which $R^1$, $R^2$ and m are as defined in claim 1 and $R^3$ is alkyl, alkanoyloxyalkyl, cycloalkanoyloxyalkyl, aryloxyalkyl or alkoxycarbonyloxyalkyl, including cycloalkyl and aryl substituted derivatives thereof, aryl or cycloalkyl, wherein said alkyl, alkanoyl and alkoxy groups contain up to 8 carbon atoms and can be branched-chain or straight-chain, said cycloalkyl groups contain from 3-7 carbon atoms and said cycloalkanoyl groups from 4-8 carbon atoms wherein both are optionally benzo-fused, and said aryl and aroyl group include optionally substituted phenyl, naphthyl and indanyl ring systems.

3. A compound as claimed in claim 2 in which $R^3$ is ethyl, indanyl, isopropyl, n-butyl, sec-butyl, t-butyl, cyclohexyl, benzyl, phenethyl, phenpropyl, acetonyl, glyceryl, pivaloyloxymethyl, 5-(4-methyl-1,3-dioxolene-2-only)methyl, cyclohexylmethyl, cyclohexylcarboxyethyl, cyclohexylacetoxyethyl, propionyloxyisobutyl, hexanoyloxyethyl, pentanoyloxyethyl, acetoxyethyl, acetoxybenzyl, pentanoyloxybenzyl, cycolhexyloxycarbonyloxyethyl, butyloxycarbonyloxyethyl, isobutyloxycarbonylethyl or ethoxycarbonyloxyethyl.

4. A compound as claimed in claim 3, wherein $R^3$ is $C_1$-$C_4$ alkyl.

**5.** A compound as claimed in anyone of the preceding claims in which $R^2$ is in the 5-position.

**6.** A compound as claimed in claim 5 in which $R^2$ is H, $C_1$-$C_4$ alkoxy or halo.

**7.** A compound as claimed in any one of the preceding claims in which $R^1$ is H or methyl.

**8.** A compound as claimed in any one of the preceding claims in which m is 4.

**9.** 5-(2-Methyl-3-[3-pyridinyl]-1H-indol-l-yl)pentanoic acid.

**10.** A pharmaceutical composition comprising a compound or salt thereof as claimed in any one of the preceding claims and a pharmaceutically acceptable diluent or carrier.

**11.** A compound or salt thereof as claimed in any one of claims 1 to 9 for use as a medicament.

**12.** The use of a compound or salt thereof as claimed in any one of claims 1 to 9 for the manufacture of a medicament for treating atherosclerosis, unstable angina or arterial re-occlusion.

European Patent Office

# EUROPEAN SEARCH REPORT

Application Number
EP 94 30 6370

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | EP-A-0 080 154 (CIBA-GEIGY AG)<br>* claims *<br>--- | 1,10 | C07D401/04<br>A61K31/44 |
| A | EP-A-0 073 663 (PFIZER LTD.)<br>* claims *<br>--- | 1,10 | |
| A | EP-A-0 069 513 (PFIZER CORPORATION)<br>* claims *<br>----- | 1,10 | |

TECHNICAL FIELDS SEARCHED (Int.Cl.6)
C07D
A61K

The present search report has been drawn up for all claims

| Place of search | Date of completion of the search | Examiner |
|---|---|---|
| THE HAGUE | 10 October 1994 | Van Bijlen, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)